(19) Europäisches Patentamt
European Patent Office
Office européen des brevets

(11) Veröffentlichungsnummer : **0 096 243**
**B1**

(12) # EUROPÄISCHE PATENTSCHRIFT

(45) Veröffentlichungstag der Patentschrift : **07.08.85**

(21) Anmeldenummer : **83104830.1**

(22) Anmeldetag : **17.05.83**

(51) Int. Cl.[4] : **C 07 C 69/02**, C 07 C 69/025, C 07 C 69/06, C 07 C 69/07, C 07 C 69/14, C 07 C 69/145, C 07 C 69/24, C 07 C 69/533, C 07 C 69/56, C 07 C 67/08, C 07 C 67/283

(54) **Cyclohex(en)ylmethanole und ihre Ester, mit Riechstoffeigenschaften.**

(30) Priorität : **03.06.82 CH 3398/82**

(43) Veröffentlichungstag der Anmeldung : **21.12.83 Patentblatt 83/51**

(45) Bekanntmachung des Hinweises auf die Patenterteilung : **07.08.85 Patentblatt 85/32**

(84) Benannte Vertragsstaaten : **CH DE FR GB LI NL**

(56) Entgegenhaltungen :
**CH-A- 520 086**
**CH-A- 616 645**
**US-A- 4 271 324**
**JZV. AKAD. S.S.S.R. OTD. CHIM., 1959, Moskau SMIT et al. Seiten 1848-1850**

(73) Patentinhaber : **L. GIVAUDAN & CIE Société Anonyme**
**CH-1214 Vernier-Genève (CH)**

(72) Erfinder : **Schenk, Hanspeter, Dr.**
**Mettelacher 3**
**CH-8126 Zumikon (CH)**

(74) Vertreter : **Urech, Peter, Dr. et al**
**Grenzacherstrasse 124 Postfach 3255**
**CH-4002 Basel (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 096 243 B1**

Jouve, 18, rue St-Denis, 75001 Paris, France

**Beschreibung**

Die Erfindung betrifft neue Riechstoffe. Es handelt sich dabei um die Verbindungen der Formel

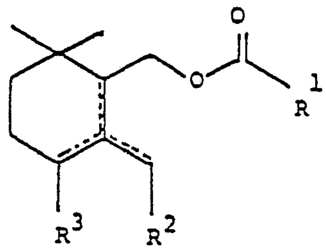

(I)

worin $R^1$ Wasserstoff, $C_{1-4}$-Alkyl oder $C_{2-4}$-Alkenyl bedeutet und $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt.

Die Formel I soll alle möglichen Stereoisomeren umfassen, also im Falle der ringungesättigten Verbindungen I insbesondere die geometrischen Isomeren der $\gamma$-Derivate und im Falle der ringgesättigten Verbindungen I insbesondere die durch die relative Lage der Substituenten am $C_1$-, $C_2$- und $C_3$-Atom von I möglichen Stereoisomeren.

Die Reste $R^1$ können geradkettig oder verzweigt sein, Beispiele sind : Methyl, Aethyl, Propyl, i-Butyl, Allyl, Methallyl, etc.

Bevorzugt sind : Wasserstoff, Methyl, Aethyl, i-Propyl, l-Propenyl, besonders bevorzugt ist Methyl.

Die Erfindung betrifft ferner ein Verfahren zur Herstellung der Verbindungen der Formel I.

Dieses Verfahren ist dadurch gekennzeichnet, dass man einen Alkohol der Formel

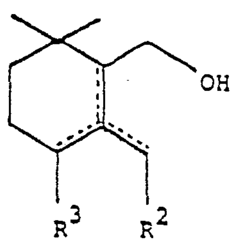

(II)

worin $R^2$ und $R^3$ und die gestrichelten Linien obige Bedeutung besitzen,
verestert, oder dass man einen Ester der Formel

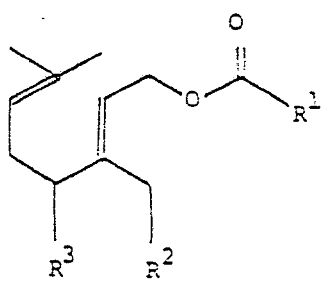

(III)

(welche Formel die beiden Doppelbindungsstereoisomeren in Stellung 2 umfassen soll)
worin $R^1$, $R^2$, $R^3$ obige Bedeutung besitzen,
cyclisiert, oder dass man eine Verbindung der allgemeinen Formel

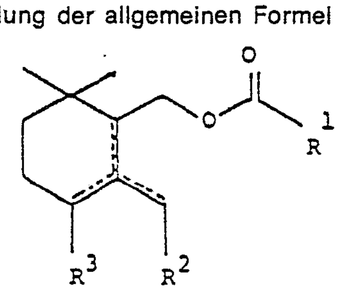

(Ia)

worin $R^1$, $R^2$ und $R^3$ obige Bedeutung besitzen, und eine der drei gestrichelt gezeichneten Linien eine zusätzliche Bindung darstellt,
katalytisch hydriert.

Der Veresterung der Alkohole II wird in an sich bekannter Weise unter Verwendung der üblichen Acylierungsmittel, also z. B. Acylhalogeniden oder Säureanhydriden durchgeführt. Bevorzugt ist die Arbeitsweise unter Verwendung der Acylhalogenide. Man arbeitet zweckmässigerweise in Anwensenheit von tertiären Amien, wie Pyridin oder Dimethylanilin.

Man kann aber auch, wie gesagt, unter Verwendung des Säureanhydrides arbeiten ; die Reaktionsbedingungen werden in diesem Fall zweckmässigerweise analog gewählt.

Die Ester I werden zweckmässig durch Destillation unter vermindertem Druck gereinigt ; sie stellen farblose bis leicht gelblich gefärbte Flüssigkeiten dar, sind unlöslich in Wasser, aber löslich in organischen Lösungsmitteln, wie z. B. Alkoholen, Aethern, Ketonen, Estern, Kohlenwasserstoffen und halogenierten Kohlenwasserstoffen.

Die Cyclisierung der Ester III kann nach den bekannten Methoden der Herstellung von Cyclogeranoylderivaten erfolgen, siehe z. B. Smit et al, Izv. Akad. S.S.S.R. Otd. chim. *1959,* 1848.

Geeingnete Cyclisierungsmittel sind anorganische und organische Protonsäuren, wie Schwefelsäure, Phosphorsäure, Methansulfonsäure, Ameisensäure, Essigsäure, etc., oder Lewissäuren wie $BF_3$, $SnCl_4$, $ZnCl_2$, etc.

Die Cyclisierung kann mit oder ohne Lösungsmittel durchgeführt werden. Geeignete Lösungsmittel sind inerte Lösungsmittel wie Hexan, Benzol, Nitromethan, etc. Die Temperatur ist nicht kritisch (Raumtemperatur, oder höhere oder niedere Temperaturen).

Die Hydrierung der Doppelbindung(en) einer Verbindung Ia, üblicherweise als Stereoisomerengemisch, erfolgt katalytisch.

Geeignete Katalysatoren für dieses Verfahren sind Edelmetallkatalysatoren, die z. B. Platin, Palladium, Ruthenium oder Rhodium enthalten.

Die Hydrierung kann mit oder ohne Lösungsmittelzusatz erfolgen ; bevorzugt sind inerte Lösungsmittel wie Aethylalkohol, Methylalkohol, Cyclohexan etc.

Die Hydrierung kann bei Temperaturen zwischen z. B. 0°-100 °C, insbesondere zwischen 15-30 °C, sowie bei Normaldruck oder auch höheren Drucken, z. B. 5-20 atü durchgeführt werden. (H.O. House, Modern Synthetic Reactions, Kapitel 1, N.A. Benjamin Inc., Menlo Park, Cal. 1972).

Nach dem erfindungsgemässen Verfahren fällt I üblicherweise als Isomerengemisch an ; in diesem Isomerengemisch überwiegt üblicherweise das (bevorzugte) α-Doppelbingungsisomere deutlich.

Die Trennung der Isomerengemische kann, falls erwünscht, auf übliche Weise, z. B. mittels präparativer Gaschromatographie erfolgen. Diese Methode dient insbesondere zur Gewinnung der reinen α-Isomeren. Die Isomeren von I unterscheiden sich in ihren organoleptischen Eigenschaften nicht grundlegend, sodass aus wirtschaftlichen Gründen insbesondere das Isomerengemisch verwendet werden kann.

Die Verbindungen I weisen besondere organoleptische Eigenschaften auf, auf Grund derer sie sich vorzüglich als Riechstoffe eignen.

Die Erfindung betrifft demgemäss auch die Verwendung der Verbindungen I als Riechstoffe.

Die Verbindungen der formel I eignen sich aufgrund ihrer natürlichen Geruchsnoten insbesondere zur Modifizierung von bekannten Kompositionen. Sie verfügen im allgemeinen über einen holzigpudrigen Charakter. Die interessante, gut haftende (Edel-) Holznote ist im allgemeinen begleitet von leicht- ambraartigen, würzigpfeffrigen Nebennoten.

So riechen z. B.

1) 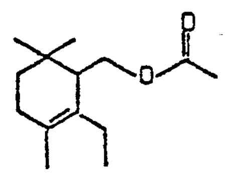

trocken, holzig, würzig-pfeffrig, ambraartig

2) 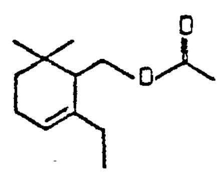 + 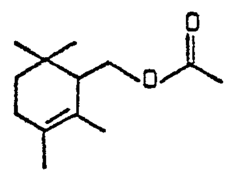

Gemisch : holzig, camphrig, ambraartig

3) 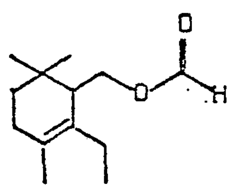

holzig, camphrig, pudrig

4) 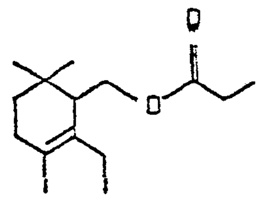

holzig, leicht säuerlich, fettig

5) 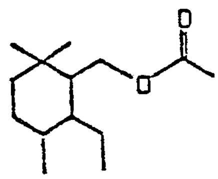

camphrig, holzig-cedrig, würzig, an Patchouli erinnernd

6) 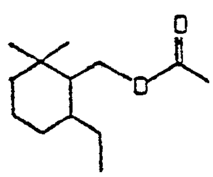 + 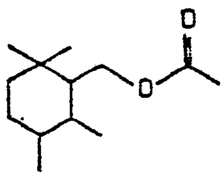

Gemisch : edle Holznote, pudrig, entfernt jononartig

7) 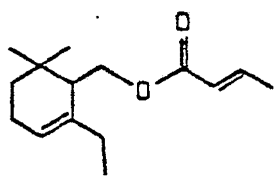 + 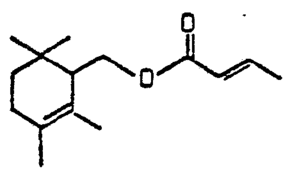

Gemisch : buttrig, im Fond pudrig.

8) 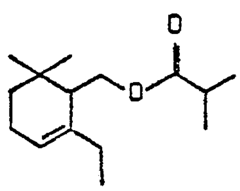 + 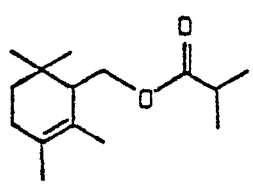

Gemisch : aldehydig, fettig, agreste (insbesondere Eucalyptus-Samen)

Die Ester I verbinden sich mit zahlreichen bekannten Riechstoffingredienten natürlichen oder synthetischen Ursprungs, wobei die Palette der natürlichen Rohstoffe sowohl leicht — als auch mittel — und schwer-flüchtige Komponenten, und diejenigen der Synthetika Vertreter aus praktisch allen Stoffklassen umfassen kann, wie dies aus der folgenden Zusammenstellung ersichtlich ist :

— Naturprodukte wie Basilikumöl, Bergamotteöl, Cedernholzöl, Corianderöl, Eichenmoos-Absolue, Elemiöl, Fichtennadelöl, Galbanumöl, Jasmin-Absolue und dessen rekonstituiertes Substitut, Patchouliöl, Petitgrainöl Paraguay, Sandelholzöl, Zitronenöl, etc.

— Alkohole, wie Citronellol, Linalool, cis-6-Nonenol, Phenyläthylalkohol, Rhodinol (Rhodinol/Citronellol-Gemisch), α-Terpineol, Zimtalkohol bzw. dessen toxikologisch unbedenkliche Substitute, etc.

— Aldehyde, wie Cyclamenaldehyd, Decanal, Hydroxycitronellal, Lilial® (p-tert. Butyl-α-methylhydrozimtaldehyd), etc.

— Ketone, wie p-Methyl-acetophenon, Methyljonon, etc.

— Ester, wie Acetessigsäureäthylester, ortho- und paratert. Butylcyclohexylacetat, Cedrylacetat, Cinnamylformiat, Geranylacetat, cis-3-Hexenylsalicylat, Maltylisobutyrat, Methyldihydrojasmonat, Myraldylacetat® ([4-(4-Methyl-3-pentenyl)-3-cyclohexen-1-yl]methylacetat), Terpenylacetat, etc.

— Lactone, wie γ-Undecalacton, etc.

— stickstoff- bzw. schwefelhaltige Verbindungen, wie Indol, p-Menthan-8-thiol-3-on etc.

— Moschusverbindungen, wie Ketonmoschus, Musk 174® (12-Oxahexadecanolid), etc.

Bemerkenswert ist ferner die Art und Weise, wie die Verbindungen I die Geruchsnoten bekannter Kompositionen abrunden und harmonisieren, ohne aber zu dominieren.

Die Verbindungen der Formel I (bzw. deren Gemische) lassen sich in weiten Grenzen einsetzen, die beispielsweise von 0,1 (Detergentien) — 30 % (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 25 %. Die mit

I hergestellten Kompositionen lassen sich für alle Arten von parfümierten Verbrauchsgütern einsetzen (Eaux de cologne, Eaux de Toilette, Extraits, Lotionen, Crèmes, Shampoos, Seifen, Salben, Puder, Zahnpasten, Mundwässer, Desodorantien, Detergentien, etc.).

Die Verbindungen I (bzw. deren gemische) können demgemäss bei der Herstellung von Kompositionen und — wie obige Zusammenstellung zeigt — unter Verwendung einer breiten Palette bekannter Riechstoffe bzw. Riechstoffgemische verwendet werden. Bei der Herstellung solcher Kompositionen können die oben angeführten bekannten Riechstoffe bzw. Riechstoffgemische nach (dem Parfümeur bekannter) Art und Weise verwendet werden, wie z. B. W.A. Poucher, Perfumes, Cosmetics, Soaps *2,* 7. Auflage, Chapman und Hall, London 1974 hervorgehend.

Die als Ausgangsmaterialien zur Herstellung der Verbindungen I dienenden Alkohole II sind ebenfalls neu und bilden einen weiteren Bestandteil der vorliegenden Erfindung. Sie bilden ebenfalls Riechstoffe ; sie wirken jedoch insgesamt schwächer und weisen camphrigere Noten als die Verbindungen I auf. Diese letzteren Verbindungen werden deshalb vor den Verbindungen II bevorzugt.

Ihre Herstellung kann wie folgt erfolgen :

a)

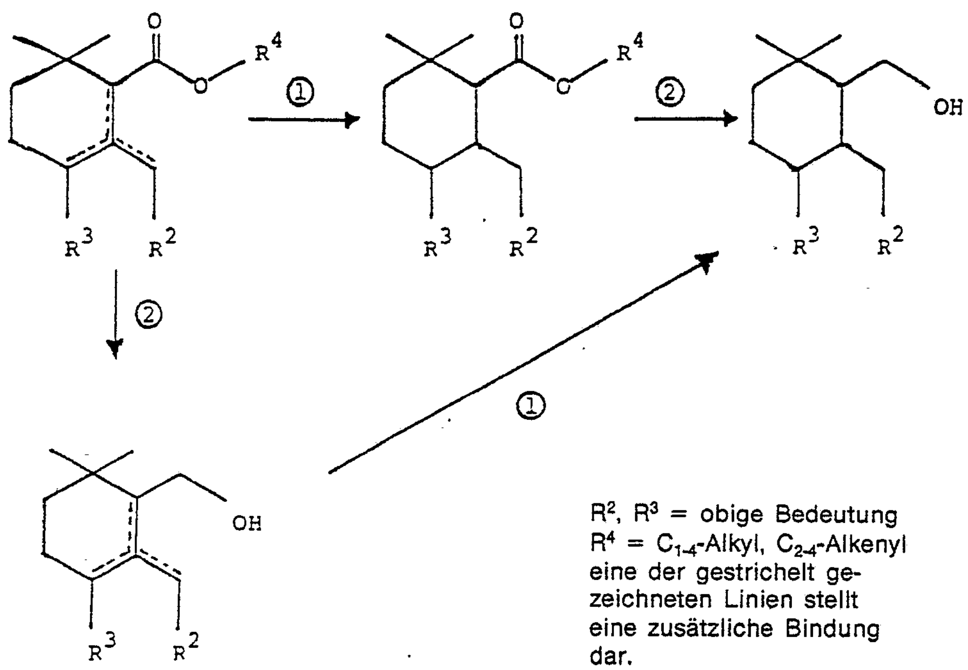


1. Katalytische Hydrierung ; unter den oben angegebenen Bedingungen

2a) Reduktion nach Bouveault-Blanc ; siehe z. B. H.O. House, Modern Synthetic Reactions, 2. Auflage, Kapitel 3, W.A. Benjamin Inc., Menlo Park, Cal. 1972.

2b) Reduktion mit Metallhydriden ; siehe z. B. H.O. House, Modern Synthetic Reactions, 2. Auflage, Kapitel 2, W.A. Benjamin Inc., Menlo Park, Cal. 1972.

## Beispiel 1

25 g (0,137 Mol) eines Alkoholgemisches, bestehend aus rund 80 % 2-Aethyl-3,6,6-trimethyl-2-cyclohexen-1-methanol (Rest : Doppelbindungsisomere), 15,4 g Essigsäureanhydrid und 13 g Pyridin werden während 15 Stunden bei 60 °C gerührt. Die Reaktionsmischung wird mit Hexan verdünnt und anschliessend mit 2N Salzsäure, 2N Natriumcarbonatlösung und schliesslich mit Salzwasser neutralgewaschen, über Natriumsulfat getrocknet und am Rotationsverdampfer von Lösungsmittel befreit. Das Rohprodukt (30,3 g) wird über eine 5 cm-Widmer-Kolonne im Hochvakuum fraktioniert destilliert. Man erhält 24,4 g (79,3 % der Theorie) eines Estergemisches vom Sdp. : 63-64 °C/0,09 mm Hg, bestehend aus rund 80 % 2-Aethyl-3,6,6-trimethyl-2-cyclohexen-1-methylacetat (Rest : Doppelbindungsisomere), $n_D^{20}$ : 1,467 5.

IR (liq.) : 1 745 $cm^{-1}$ (C = O, Ester)

NMR ($CDCl_3$) : 0,87 / 0,94 } je s (3H) $C_6<^{CH_3}_{CH_3}$

0,93 t/7 (3H) $C_2$—$CH_2$—$CH_3$
1,60 s (3H) $C_3$—$CH_3$
2,00 s (3H) $\overset{O}{\overset{\|}{C}}$—$CH_3$
4,01 d/5 (2H) $C_1$—$CH_2$—O—
MS : 224 (M, 1), 43 (100), 164 (79), 149 (78), 93 (56), 12 (43).

Das als Ausgangsmaterial verwendete Alkoholgemisch wird wie folgt dargestellt : 9,7 g (0,256 Mol) Lithiumaluminiumhydrid werden in 600 ml absolutem Diäthyläther vorgelegt. Die Suspension wird auf 10 °C abgekühlt. Bei dieser Temperatur wird eine Lösung von 100 g eines Estergemisches, enthaltend rund 80 % 2-Aethyl-3,6,6-trimethyl-2-cyclohexen-1-carbonsäureäthylester (Rest : Doppelbindungsisomere) in 150 ml absolutem Diäthyläther innert 2 Stunden zugetropft. Nach dem Zutropfen lässt man 12 Stunden bei Raumtemperatur ausreagieren. Das Reaktionsgemisch wird auf 0 °C abgekühlt und unter Zutropfen mit 100 ml einer eiskalten gesättigten Ammoniumchlorid-Lösung vorsichtig versetzt, so dass die Temperatur nicht über 10 °C steigt. Im Scheidetrichter wird die organische Phase abgetrennt und die Wasserphase noch zweimal mit Aether extrahiert. Die vereinigten organischen Phasen werden neutralgewaschen, über Natriumsulfat getrocknet und am Rotationsverdampfer eingedampft. Das Rohprodukt (79,6 g) wird über eine 10 cm-Widmer-Kolonne im Hochvakuum fraktioniert destilliert. Man erhält 69,8 g (85,9 % der Theorie) eines Alkoholgemisches vom Sdp. : 68-70 °C/0,06 mm Hg, bestehend aus rund 80 % 2-Aethyl-3,6,6-trimethyl-2-cyclohexen-1-methanol (Rest : Doppelbindungsisomere) ; $n_D^{20}$ : 1,483 9.

IR (liq.) : 3 600-3 100 $cm^{-1}$ (OH)
NMR ($CDCl_3$) : 0,88 / 1,07 je s (3H) $C_6$<$CH_3$ / $CH_3$
0,98 t/7 (3H) $CH_2$—$CH_3$
1,70 s (3H) $C_3$-$CH_3$
3,70 d/5 (2H) $C_1$—$CH_2$—OH
MS : 182 (M, 10), 141 (100), 95 (43), 109 (38), 41 (27), 55 (18).

Beispiel 2

Analog dem im Beispiel 1 beschriebenen Verfahren werden aus 16,8 g (0,1 Mol) eines Alkoholgemisches, enthaltend rund 20 % 2,3,6,6-Tetramethyl-2-cyclohexen-1-methanol (einschliesslich Doppelbindungsisomere. Die Isomerengemische enthalten mehr als 75 % des 2-Cyclohexen-Derivates) und 80 % 2-Aethyl-6,6-dimethyl-2-cyclohexen-1-methanol (einschliesslich Doppelbindungsisomere. Die Isomerengemische enthalten mehr als 75 % des 2-Cyclohexen-Derivates) 13,9 g (66.2 % der Theorie) eines Estergemisches, bestehend aus rund 20 % 2,3,6,6-Tetramethyl-2-cyclohexen-1-methylacetat (einschliesslich Doppelbindungsisomere. Die Isomerengemische enthalten mehr als 75 % des 2-Cyclohexen-Derivates) und 80 % 2-Aethyl-6,6-dimethyl-2-cyclohexen-1-methylacetat (einschliesslich Doppelbindungsisomere. Die Isomerengemische enthalten mehr als 75 % des 2-Cyclohexen-Derivates) erhalten. Sdp. 59-60 °C/0,09 mm Hg ; $n_D^{20}$ : 1,466 1.

Das als Ausgangsmaterial verwendete Alkoholgemisch erhält man analog dem im Beispiel 1 beschriebenen Verfahren aus einem Estergemisch, bestehend aus rund 20 % 2,3,6,6-Tetramethyl-2-cyclohexen-1-carbonsäureäthylester (einschliesslich Doppelbindungsisomere. Die Isomerengemische enthalten mehr als 75 % des 2-Cyclohexen-Derivates) und 80 % 2-Aethyl-6,6-dimethyl-2-cyclohexen-1-carbonsäureäthylester (einschliesslich Doppelbindungsisomere. Die Isomerengemische enthalten mehr als 75 % des 2-Cyclohexen-Derivates) durch Reduktion mit Lithiumaluminiumhydrid in einer Ausbeute von 91,6 % der Theorie. Sdp. : 48-50 °C/0,04 mm Hg ; $n_D^{20}$ : 1,485 5.

Beispiel 3

10,5 g (0,228 Mol) Ameisensäure werden zu 18,6 g (0,182 Mol) Essigsäureanhydrid zugetropft. Das Gemisch wird während 15 Minuten auf 50 °C erwärmt und anschliessend auf 0 °C abgekühlt. Bei dieser Temperatur werden 25 g (0,137 Mol) eines Alkoholgemisches, enthaltend rund 80 % 2-Aethyl-3,6,6-trimethyl-2-cyclohexen-1-methanol (Rest : Doppelbindungsisomere) zugetropft. Man lässt 12 Stunden bei einer Temperatur von 0-5 °C ausreagieren. Das Reaktionsgemisch wird in 50 ml Hexan aufgenommen, mit Wasser, 2N Sodalösung und wieder mit Wasser neutralgewaschen, über Natriumsulfat getrocknet und am Rotationsverdampfer eingeengt. Das Rohprodukt (27,5 g) wird über eine 10 cm-Widmer-Kolonne im Hochvakuum fraktioniert destilliert. Man erhält 21,8 g (75,6 % der Theorie) eines Estergemisches, enthaltend rund 80 % 2-Aethyl-3,6,6-trimethyl-2-cyclohexen-1-methylformiat (Rest : Doppelbindungsisomere). Sdp. 52-54 °C/0,04 mm Hg ; $n_D^{20}$ : 1,472 1.

IR (liq.) : 1 730 $cm^{-1}$

NMR ($CDCl_3$) :
0,90 / 0,99 je s (3H) $C_6 \langle ^{CH_3}_{CH_3}$
0,95 t/7 (3H) $C_2—CH_2—CH_3$
1,66 s (3H) $C_3—CH_3$
4,15 d/5 (2H) $C_1—CH_2—O—$
8,02 s (1H) $—O\overset{O}{\overset{\|}{C}}—H$

MS : 151 (100), 41 (98), 93 (96), 164 (93), 95 (88), 149 (86).

Das als Ausgangsmaterial verwendete Alkoholgemisch wird gemäss Verfahren des Beispiels 1 erhalten.

## Beispiel 4

25 g (0,137 Mol) eines Alkoholgemisches, das zu rund 80 % aus 2-Aethyl-3,6,6-trimethyl-2-cyclohexen-1-methanol besteht (Rest : Doppelbindungsisomere), 19,6 g (0,151 Mol) Propionsäureanhydrid und 13,0 g Pyridin werden 12 Stunden bei 60 °C gerührt. Das Reaktionsgemisch wird mit Hexan verdünnt und anschliessend mit 2N Salzsäure, 2N Sodalösung und Salzwasser neutralgewaschen, über Natriumsulfat getrocknet und am Rotationsverdampfer eingedampft. Das Rohprodukt (30,4 g) wird über eine 10 cm-Widmer-Kolonne im Hochvakuum fraktioniert destilliert. Man erhält 26,2 g (80.1 % der Theorie) eines Estergemisches, bestehend aus rund 80 % 2-Aethyl-3,6,6-trimethyl-2-cyclohexen-1-methyl-propionat (Rest : Doppelbindungsisomere). Sdp. 75-76 °C/0,05 mm Hg ; $n_D^{20}$ : 1,467 2.

IR (liq.) : 1 740 $cm^{-1}$

NMR ($CDCl_3$) :
0,87 / 0,97 je s (3H) $C_6 \langle ^{CH_3}_{CH_3}$
0,92 t/7 (3H) $C_2—CH_2—CH_3$
1,59 s (3H) $C_3—CH_3$
4,03 d/s (2) $C_2—CH_2—O—$

MS : 164 (100), 149 (99), 29 (84), 57 (81), 135 (55), 151 (51).

Das als Ausgangsmaterial verwendete Alkoholgemisch wird gemäss Verfahren des Beispiels 1 erhalten.

## Beispiel 5

Ein Gemisch von 10 g (54,3 Millimol) 2-Aethyl-3,6,6-trimethyl-cyclohexan-1-methanol (4 Stereoisomere), 15 ml Essigsäureanhydrid und 100 ml Pyridin wird 12 Stunden bei Raumtemperatur stehen gelassen. Ueberschüssiges Pyridin und Essigsäureanhydrid werden am Rotationsverdampfer bei einer Badtemperatur von rund 50 °C im Wasserstrahlvakuum abdestilliert. Der Rückstand wird in Hexan aufgenommen, mit 2N Salzsäure, gesättigter Natriumbicarbonatlösung und Wasser neutralgewaschen, über Natriumsulfat getrocknet und eingedampft. Das Rohprodukt (11,4 g) wird über eine 10 cm-Vigreux-Kolonne im Hochvakuum fraktioniert destilliert. Dabei erhält man 7,0 g (57,0 % der Theorie) 2-Aethyl-3,6,6-trimethyl-cyclohexan-1-methyl-acetat (4 Stereoisomere) vom Sdp. : 65-67 °C/0,08 mm Hg ; $n_D^{20}$ : 1,459 9.

IR (liq.) : 1 740 $cm^{-1}$ (C = O, Ester)
MS : 43 (100), 55 (75), 59 (58), 81 (50), 95 (49), 41 (48).

Das als Ausgangsmaterial verwendete 2-Aethyl-3,6,6-trimethyl-cyclohexan-1-methanol (4 Stereoisomere) erhält man analog dem im Beispiel 1 beschriebenen Verfahren aus dem 2-Aethyl-3,6,6-trimethyl-cyclohexan-1-carbonsäureäthylester (4 Stereoisomere) durch Reduktion mit Lithiumaluminiumhydrid in 97,5 %iger Ausbeute.
Sdp. : 75 °/0,05 mm Hg ; $n_D^{20}$ : 1,476 3.

Für die Darstellung des 2-Aethyl-3,6,6-trimethyl-cyclohexan-1-carbonsäureäthylesters wird wie folgt verfahren :

22,4 g (0,1 Mol) eines Estergemisches bestehend aus ca. 80 % 2-Aethyl-3,6,6-trimethyl-2-cyclohexen-1-carbonsäureäthylester (Rest : Doppelbindungsisomere) wird in 250 ml absolutem Aethylalkohol gelöst und unter Zugabe von 800 mg Palladium (10 % auf Kohle) im Autoklaven bei 10 bar und 60 °C während 24 Stunden hydriert. Der Katalysator wird über Celite abfiltriert, mit wenig Aethylalkohol nachgewaschen und das Lösungsmittel am Rotationsverdampfer abdestilliert.

Das Rohprodukt (21,8 g) wird über eine 5 cm-Widmer-Kolonne im Hochvakuum fraktioniert destilliert. Man erhält 18,0 g (79,6 % d. Th.) eines Gemisches vom Siedepunkt 80-81°/0,15 mm Hg ; $n_D^{20}$ : 1,452 7. Im

Kapillargaschromatogramm (50 m × 0,31 mm i. D., Ucon HB 5100, 140 °C isotherm, Heliumfluss 2,5 ml/Min. Splitverhältnis 1 : 30) sind 4 Peaks ersichtlich, mit folgenden prozentualen Anteilen am Gesamtgemisch (geordnet nach steigender Retentionszeit) :

| | |
|---|---|
| $P_1$ | 42,4 % |
| $P_2$ | 34,9 % |
| $P_3$ | 16,5 % |
| $P_4$ | 6,2 % |

Peaks 1, 2, 3, 4 stellen die 4 möglichen Stereoisomeren des 2-Aethyl-3,6,6-trimethyl-cyclohexan-1-carbonsäureäthylesters dar.

Beispiel 6

Ein Gemisch von 10 g (58,8 Millimol) ca. 80 % 2-Aethyl-6,6-dimethyl-cyclohexan-1-methanol (2 Stereoisomere) und ca. 20 % 2,3,6,6-Tetramethyl-cyclohexan-1-methanol (4 Stereoisomere) wird zusammen mit 15 ml Essigsäureanhydrid und 100 ml Pyridin 12 Stunden bei Raumtemperatur stehen gelassen. Ueberschüssiges Pyridin und Essigsäureanhydrid werden am Rotationsverdampfer bei einer Badtemperatur von rund 50 °C im Wasserstrahlvakuum abdestilliert. Der Rückstand wird in Hexan aufgenommen, mit 2N Salzsäure, gesättigter Natriumbicarbonatlösung und Wasser neutralgewaschen, über Natriumsulfat getrocknet und eingedampft. Das Rohprodukt (10,9 g) wird über eine 5 cm-Vigreux-Kolonne im Hochvakuum fraktioniert destilliert. Dabei erhält man 6,8 g (54,5 % der Theorie) eines Gemisches, bestehend aus rund 80 % 2-Aethyl-6,6-dimethyl-cyclohexan-1-methylacetat (2 Stereoisomere) und 20 % 2,3,6,6-Tetramethyl-cyclohexan-1-methylacetat (4 Stereoisomere) vom Sdp. : 67-68 °C/0,06 mm Hg ; $n_D^{20}$ : 1,457 1.

Das als Ausgangsmaterial verwendete Gemisch erhält man analog dem in Beispiel 1 beschriebenen Verfahren aus einem Gemisch, bestehend aus rund 80 % 2-Methyl-6,6-dimethyl-cyclohexan-1-carbonsäureäthylester und 20 % 2,3,6,6-Tetramethyl-cyclohexan-1-carbonsäureäthylester durch Reduktion mit Lithiumaluminiumhydrid in 71,2 % der Theorie. Sdp. : 72 °C/0,05 mm Hg : $n_D^{20}$ : 1,474 6.

Für die Darstellung des obigen Aethylestergemisches wird wie folgt verfahren :

Zu einer gekühlten Lösung von 5,8 g (0,252 gAtom) Natrium in 130 ml absoluten Aethanol wird bei einer Temperatur von 5-10 °C eine Lösung von 30 g (0,214 Mol) eines Ketongemisches, bestehend aus 20 % 3,6-Dimethyl-5-hepten-2-on und 80 % 7-Methyl-6-octen-3-on und 62,4 g (0,278 Mol) Phosphonoessigsäuretriäthylester in 130 ml absolutem Toluol getropft. Anschliessend lässt man das Reaktionsgemisch Raumtemperatur annehmen und 12 Stunden ausreagieren. Das Reaktionsgemisch wird auf Eiswasser gegossen und 3 mal mit Hexan extrahiert. Die vereinigten Hexanlösungen werden mit Kochsalzlösung neutralgewaschen, über Natriumsulfat getrocknet und eingedampft. Das Rohprodukt (43 g) wird im Hochvakuum über eine 10 cm-Widmer-Kolonne fraktioniert destilliert. Man erhält 28,9 g (64,3 %) eines Gemisches vom Siedepunkt : 58-61 °C/0,02 mm Hg ; $n_D^{20}$ : 1,470 8. Das Gemisch besteht aus 20 % c, t-3,4,7-Trimethyl-2,6-octadiensäureäthylester und 80 % c, t-3-Aethyl-7-methyl-2,6-octadiensäureäthylester.

228 ml Ameisensäure werden auf 0-5 °C abgekühlt. Bei dieser Temperatur werden 12 ml konz. Schwefelsäure zugegeben und anschliessend wird das Gemisch 1 Stunde gerührt. Zu diesem erhaltenen Säuregemisch werden bei + 5 °C 24 g des obigen Estergemisches, bestehend aus 20 % c, t-3,4,7-Trimethyl-2,6-octadiensäureäthylester und 80 % c, t-3-Aethyl-7-methyl-2,6-octadiensäureäthylester vorsichtig zugetropft. Nach beendeter Zugabe lässt man das Gemisch Raumtemperatur annehmen und rührt es bei dieser Temperatur 1 Stunde weiter. Das Reaktionsgemisch wird auf Eis gegossen und 3 mal mit Hexan extrahiert. Die vereinigten Hexanlösungen werden mit Wasser (1 mal), mit Natriumbicarbonatlösung (2 mal) und schliesslich mit Wasser (2 mal) neutralgewaschen, über Natriumsulfat getrocknet und eingedampft. Das Rohprodukt (22,5 g) wird im Hochvakuum an einer 10 cm-Widmer-Kolonne fraktioniert destilliert. Man erhält 17 g (70,8 %) eines Estergemisches, bestehend aus rund 20 % 2,3,6,6-Tetramethyl-2-cyclohexen-1-carbonsäureäthylester, 14 % c, t-2-Aethyliden-6,6-dimethyl-cyclohexan-1-carbonsäureäthylester und 65 % 2-Aethyl-6,6-dimethyl-2-cyclohexen-1-carbonsäureäthylester vom Siedepunkt : 102 °C/6 mm Hg ; $n_{D20}$ : 1,462 6.

30 g dieses Estergemisches werden in 300 ml absolutem Aethylalkohol gelöst und unter Zugabe von 600 mg Palladium (10 % auf Kohle) unter gutem Rühren bei Normaldruck hydriert. Nach 24 Stunden sind 96,9 % der theoretischen Wasserstoffmenge aufgenommen. Der Katalysator wird über Celite abfiltriert, mit wenig Aethanol nachgewaschen und das Lösungsmittel am Rotationsverdampfer abdestilliert.

Das Rohprodukt (29,8 g) wird über eine 10 cm-Widmer-Kolonne im Hochvakuum fraktioniert destilliert. Man erhält 28 g (92,4 % d. Th.) eines Gemisches vom Siedepunt 42-55 °C/0,05 mm Hg. Gemäss Gaschromatogramm [Glaskapillarsäure (50 m × 0,3 mm I. D.) mit Ucon HB 5100 als stationärer Phase, 140° isotherm, Heliumzufluss 2,5 ml/Min.] ergibt sich im wesentlichen die folgende Produktezusammensetzung : 43,6 % cis-2-Aethyl-6,6-dimethylcyclohexan-1-carbonsäureäthylester, 36,4 % trans-2-Aethyl-6,6-dimethylcyclohexan-1-carbonsäureäthylester und 20,0 % 2,3,6,6,-Tetramethylcyclohexan-1-carbonsäureäthylester (verschiedene Stereoisomeren, u. a. ca. 4,1 % 1,2 cis-2,3-trans-2,3,6,6-Tetramethyl-1-cyclohexancarbonsäureäthylester und ca. 9,1 % 1,2 trans-2,3-trans-2,3,6,6-Tetramethyl-1-cyclohexancarbonsäureäthylester). Das Isomerengemisch wurde mittels präparativer Gaschromatographie aufgetrennt. Die Hauptpeaks zeigten die folgenden spektroskopischen Daten :

cis-2-Aethyl-6,6-dimethyl-cyclohexan-1-carbonsäureäthylester

IR (liq.) : 1 735 $cm^{-1}$ — $^1$H-NMR (360 MHz, $CDCl_3$) :

| | | | | | | | |
|---|---|---|---|---|---|---|---|
| 0,89 | s | (3H) | 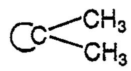 | 1,25 | t/7 | (3H) | 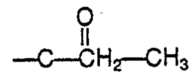 |
| 0,91 | t/7 | (3H) | $—CH_2—CH_3$ | 2,31 | d/4 | (1H) | 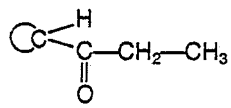 |
| 0,99 | s | (3H) | 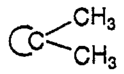 | 4,09 | AB-Teil von $ABX_3$ | (2H) | 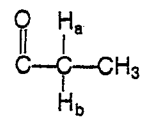 |

trans-2-Aethyl-6,6-dimethyl-cyclohexan-1-carbonsäureäthylester

IR (liq.) : 1 735 $cm^{-1}$ — $^1$H-NMR (360 MHz, $CDCl_3$) :

| | | | | | | | |
|---|---|---|---|---|---|---|---|
| 0,86 | t/7 | (3H) | $—CH_2—CH_3$ | 1,26 | t/7 | (3H) | 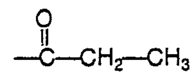 |
| 0,93 | s | (3H) | 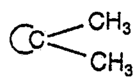 | 1,89 | d/11,5 | (1H) | 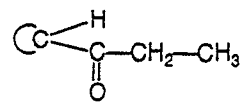 |
| 0,97 | s | (3H) | 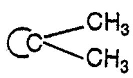 | 4,13 | q/7 | (2H) | 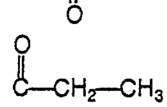 |

1,2-cis, 2,3-trans, 2,3,6,6-Tetramethyl-1-cyclohexancarbonsäureäthylester

IR (liq.) : 1 725 $cm^{-1}$ — $^1$H-NMR (360 MHz, $CDCl_3$) :

| | | | | | | | |
|---|---|---|---|---|---|---|---|
| 0,86 | d/7 | (3H) | $C_3—CH_3$ | 1,40-1,60 | m | (2H) | u. a. $C_2$-$H_{ax}$ |
| 0,87 | d/t | (3H) | $C_2—CH_3$ | 1,68-1,81 | m | (1H) | $C_3—H_{ax}$ |
| 0,89 | s | (3H) | 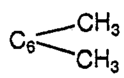 | 1,84-1,95 | m | (1H) | $C_5—H_{ax}$ |
| 0,99 | s | (3H) | | 2,22 | d/s | (1H) | $C_1—H_{eq}$ |
| 1,25 | t/7 | (3H) | 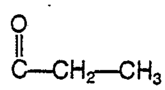 | 4,10 | q/7 | (2H) | 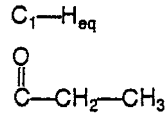 |

1,2-trans, 2,3-trans-2,3,6,6,-Tetramethyl-1-cyclohexancarbonsäureäthylester

IR (liq.) : 1 725 $cm^{-1}$ — $^1$H-NMR (360 MHz, $CDCl_3$) :

| | | | | | | | |
|---|---|---|---|---|---|---|---|
| 0,84 | d/6 | (3H) | $C_3—CH_3$ | 1,26 | t/7 | (3H) | 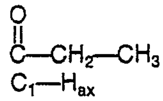 |
| 0,925 | s | (3H) | $C_6—CH_3$ | 1,87 | d/7 | (1H) | $C_1—H_{ax}$ |
| 0,93 | d/6 | (3H) | $C_2—CH_3$ | 4,14 | q/7 | (2H) | 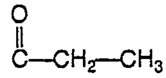 |
| 0,95 | s | (3H) | $C_6—CH_3$ | | | | |

## Beispiel 7

Analog dem im Beispiel 1 beschriebenen Verfahren (aber unter Verwendung von Crotonsäureanhydrid) werden aus 14,0 g (0,083 Mol) eines Alkoholgemisches, bestehend aus rund 20 % 2,3,6,6-Tetramethyl-2-cyclohexen-1-methanol (einschliesslich Doppelbindungsisomere. Die Isomerengemische enthalten mehr als 75 % der 2-Cyclohexen-Verbindung) und 80 % 2-Aethyl-6,6-dimethyl-2-cyclohexen-1-

methanol (einschliesslich Doppelbindungsisomere. Die Isomerengemische enthalten mehr als 75 % der 2-Cyclohexen-Verbindung) 15,4 g (78,3 % der Theorie) eines Estergemisches, bestehend aus rund 20 % 2,3,6,6-Tetramethyl-2-cyclohexen-1-methyl-crotonat (einschliesslich Doppelbindungsisomere. Die Isomerengemische enthalten mehr als 75 % der 2-Cyclohexen-Verbindung) und 80 % 2-Aethyl-6,6-dimethyl-2-cyclohexan-1-methyl-crotonat (einschliesslich Doppelbindungsisomere. Die Isomerengemische enthalten mehr als 75 % der 2-Cyclohexen-Verbindung) erhalten. Sdp. : 83-85 °C/0,06 mm Hg ; $n_D^{20}$ : 1,482 6.

Das als Ausgangsmaterial verwendete Alkoholgemisch erhält man analog dem im Beispiel 1 beschriebenen Verfahren aus einem Estergemisch, bestehend aus rund 20 % 2,3,6,6-Tetramethyl-2-cyclohexen-1-carbonsäureäthylester (einschliesslich Doppelbindungsisomere. Die Isomerengemische enthalten mehr als 75 % der 2-Cyclohexen-Verbindung) und 80 % 2-Aethyl-6,6-dimethyl-2-cyclohexen-1-carbonsäureäthylester (einschliesslich Doppelbindungsisomere. Die Isomerengemische enthalten mehr als 75 % der 2-Cyclohexen-Verbindung) durch Reduktion mit Lithiumaluminiumhydrid in einer Ausbeute von 91,6 % der Theorie. Sdp. : 48-50 °C/0.04 mm Hg ; $n_D^{20}$ : 1,485 5.

## Beispiel 8

### Parfümerie-Base Richtung Chypre

| | Gewichtsteile |
|---|---|
| Methyl-1-methylcyclododecyläther | 200 |
| Bergamotteöl | 150 |
| Hydroxycitronellal | 100 |
| Fichtenöl Pumilion | 80 |
| Citronellol | 80 |
| Petitgrainöl | 60 |
| Musc 174$^R$ Giv (12-Oxahexadecanolid) | 60 |
| Corianderöl | 40 |
| Galbanumöl | 40 |
| Zedernholzöl | 40 |
| Patchouliöl | 40 |
| Zitronenöl | 40 |
| Elemiöl | 10 |
| Eichenmoos entfärbt | 20 |
| | 960 |

Ein Zusatz von 40 Teilen des Estergemisches des Beispiels 1 wirkt sich auf die Geruchseigenschaften obiger Base sehr harmonisierend aus. Die in der usprünglichen Base etwas herbe Note des Elemi-Zedernholz-Komplex wird in vorteilhafter Weise mit dem blumigen Gemisch Hydroxycitronellol-Citronellol verbunden, so dass dieses in der resultierenden Komposition dominiert.

Gibt man zu der vorstehenden Chypre-Grundbase 40 Teile des Esters des Beispiels 5, so gewinnt die Base an Fülle und Wärme. Ueberraschend ist der zusätzlich auftretende Frische-Eindruck, der die neue Base für Seifen- und Deodorantienparfümierungen geeignet macht.

## Beispiel 9

### Parfümerie-Base in Richtung Melone

| | Gewichtsteile |
|---|---|
| Myraldylacetat$^R$ Giv ([4-(4-Methyl-3-pentenyl)-3-cyclohexen-1-yl]methylacetat) | 140 |
| Hexenylsalicylat | 80 |
| Methyldihydrojasmonat | 60 |
| Aethyl-acetyl-acetat | 60 |
| Cyclamenaldehyd | 50 |
| ortho-tert. Butyl-cyclohexylacetat | 50 |
| Lilial$^R$ Giv (p-tert. Butyl-α-methylhydrozimtaldehyd) | 10 |
| Rhodinol | 5 |
| Eugenol | 5 |
| Maltylisobutyrat (10 % in DPG) | 5 |
| Acetanisol | 5 |
| Cis-6-Nonenol (10 % in DPG) | 5 |
| Dipropylenglykol (DPG) | 425 |
| | 900 |

Durch Zusatz von 100 Teilen des Estergemisches des Beispiels 1 wird die ursprünglich grün-fettige Melone in Richtung einer saftig-fruchtigen, frischen Melone verändert. Die neue Base wirkt ausgesprochen natürlich. Dieser frische Melonencharakter stellt das angestrebte Ziel dieser Komposition dar.

Beispiel 10

Holzige Base

| | Gewichtsteile |
|---|---|
| Bergamotteöl | 200 |
| Patchouliöl | 200 |
| Sandelholzöl | 200 |
| Cedrylacetat | 100 |
| Methyldihydrojasmonat | 70 |
| Methyljonon | 50 |
| p-tert. Butylcyclohexyl-2-acetat | 50 |
| Basilikumöl | 30 |
| | 900 |

Der Zusatz von 100 Teilen des Estergemisches des Beispiels 1 zeitigt einen äusserst überraschenden Effekt. Die Evaluation im frisch getauchten Zustand zeigt, dass in der Holzkomposition plötzlich eine frische, ländlich-krautige Cologne-Note überwiegt. Ebenso unerwartet verhält sich die Fondnote der neuen Komposition : die neue Substanz vermittelt dem Fond eine ausgeprägte Vetiver-Note, obwohl Vetiveröl nicht Bestandteil dieser Komposition ist.

Beispiel 11

Allgemein-blumige Parfümerie-Base

| | Gewichtsteile |
|---|---|
| Terpineol | 260 |
| Hydroxycitronellal | 220 |
| Zimtalkoholsubstitut | 120 |
| Phenyläthylalkohol | 100 |
| Cinnamylformiat | 20 |
| Linalool | 15 |
| Terpenylacetat | 10 |
| Keton-moschus | 10 |
| Geranylacetat | 10 |
| Jasmin synth. | 10 |
| Eugenol | 5 |
| Indol (10 % in DPG) | 5 |
| C-10-Aldehyd (10 % in DPG) | 5 |
| p-Methylacetophenon | 5 |
| Undecalacton | 5 |
| | 800 |

Ein Zusatz von 200 Teilen des Estergemisches des Beispiels 6 bringt dieser allgemein blumigen Base Richtung Flieder eine ausgeprägte Hyacinthen-Note. Dies ist umso erstaunlicher, als das neue Estergemisch keine Blumennote aufweist.

**Patentansprüche**

1. Verbindungen der allgemeinen Formel

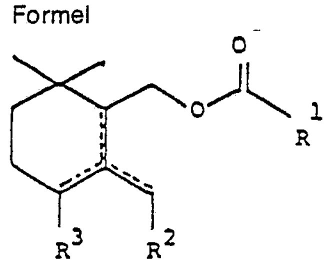

(I)

worin $R^1$ Wasserstoff, $C_{1-4}$-Alkyl oder $C_{2-4}$-Alkenyl bedeutet und $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt.

2. 2-Aethyl-3,6,6-trimethyl-2-cyclohexen-1-methylacetat.

3. Gemisch von 2-Aethyl-6,6-dimethyl-2-cyclohexen-1-methylacetat und 2,3,6,6-Tetramethyl-2-cyclohexen-1-methylacetat.

4. Verbindungen der allgemeinen Formel

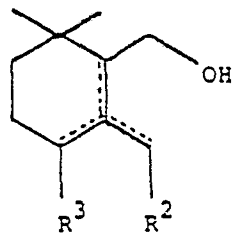

(II)

worin $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt.

5. Verfahren zur Herstellung von Verbindungen der Formel

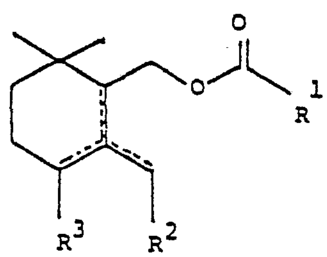

(I)

worin $R^1$ Wasserstoff, $C_{1-4}$-Alkyl oder $C_{2-4}$-Alkenyl bedeutet und $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt,
dadurch gekennzeichnet, dass man

a) einen Alkohol der Formel

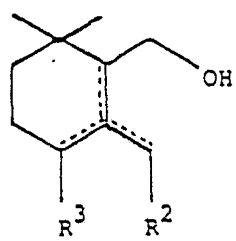

(II)

worin $R^2$ und $R^3$ und die gestrichelten Linien obige Bedeutung besitzen,
verestert oder dass man

b) einen Ester der Formel

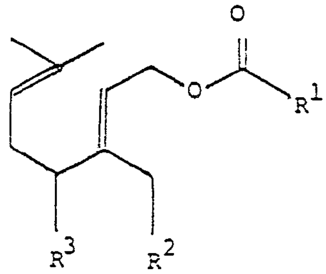

(III)

worin $R^1$, $R^2$, $R^3$ obige Bedeutung besitzen,
cyclisiert, oder dass man

c) eine Verbindung der allgemeinen Formel

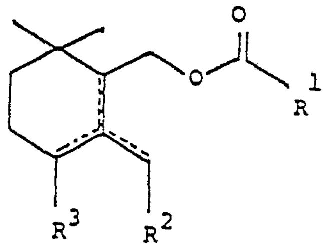

(Ia)

worin $R^1$, $R^2$ und $R^3$ obige Bedeutung besitzen, und eine der drei gestrichelt gezeichneten Linien eine zusätzliche Bindung darstellt,
katalytisch hydriert.

6. Riechstoffkomposition, gekennzeichnet durch einen Gehalt an einer Verbindung der allgemeinen Formel

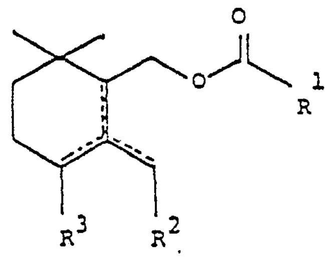

(I)

worin $R^1$ Wasserstoff, $C_{1-4}$-Alkyl oder $C_{2-4}$-Alkenyl bedeutet und $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt.

7. Riechstoffkomposition, gekennzeichnet durch einen Gehalt an 2-Aethyl-3,6,6-trimethyl-2-cyclohexen-1-methylacetat.

8. Riechstoffkomposition, gekennzeichnet durch einen Gehalt an einem Gemisch von 2-Aethyl-6,6-dimethyl-2-cyclohexen-1-methylacetat und 2,3,6,6-Tetramethyl-2-cyclohexen-1-methylacetat.

9. Riechstoffkomposition, gekennzeichnet durch einen Gehalt an einer Verbindung der allgemeinen Formel

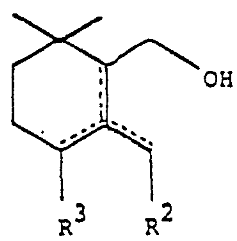

(II)

worin $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt.

10. Verwendung von Verbindungen der allgemeinen Formel

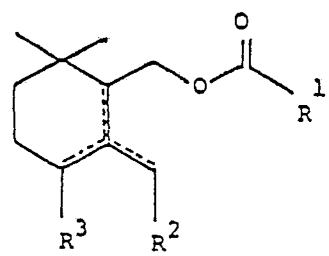

(I)

worin $R^1$ Wasserstoff, $C_{1-4}$-Alkyl oder $C_{2-4}$-Alkenyl bedeutet und $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt,
als Riechstoffe bzw. als Modifikatoren von Riechstoffkompositionen.

11. Verwendung der Verbindungen der allgemeinen Formel

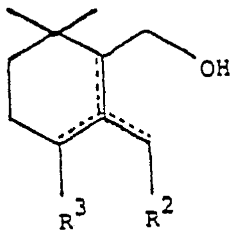

(II)

worin $R^2$ und $R^3$ Wasserstoff oder Methyl darstellen, wobei aber nicht beide Reste $R^2$ und $R^3$ gleichzeitig Wasserstoff darstellen, und eine der drei gestrichelt gezeichneten Linien eine fakultative zusätzliche Bindung darstellt,
als Riechstoffe bzw. als Modifikatoren von Riechstoffkompositionen.

**Claims**

1. Compounds of the general formula

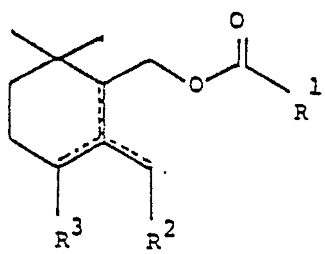

(I)

wherein $R^1$ represents hydrogen, $C_{1-4}$-alkyl or $C_{2-4}$-alkenyl and $R^2$ and $R^3$ represent hydrogen or methyl, but wherein both residues $R^2$ and $R^3$ do not simultaneously represent hydrogen, and one of the three lines depicted dotwise represents an optional additional bond.

2. 2-Ethyl-3,6,6-trimethyl-2-cyclohexene-1-methyl acetate.

3. A mixture of 2-ethyl-6,6-dimethyl-2-cyclohexene-1-methyl acetate and 2,3,6,6-tetramethyl-2-cyclohexene-1-methyl acetate.

4. Compounds of the general formula

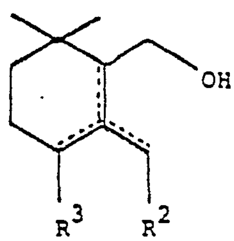

(II)

wherein $R^2$ and $R^3$ represent hydrogen or methyl, but wherein both residues $R^2$ and $R^3$ do not simultaneously represent hydrogen, and one of the three lines depicted dotwise represents an optional additional bond.

5. A process for the manufacture of compounds of the formula

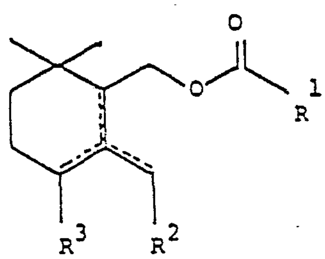

(I)

wherein $R^1$ represents hydrogen, $C_{1-4}$-alkyl or $C_{2-4}$-alkenyl and $R^2$ and $R^3$ represent hydrogen or methyl, but wherein both residues $R^2$ and $R^3$ do not simultaneously represent hydrogen, and one of the three lines depicted dotwise represents an optional additional bond,
characterized by

a) esterifying an alcohol of the formula

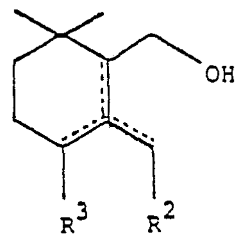

(II)

wherein $R^2$ and $R^3$ and the dotted lines have the above significance, or

b) cyclizing an ester of the formula

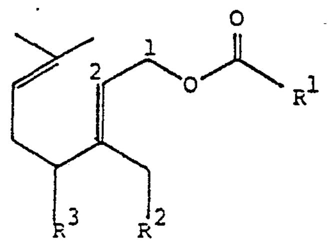

(III)

wherein $R^1$, $R^2$, $R^3$ have the above significance, or

c) catalytically hydrogenating a compound of the general formula

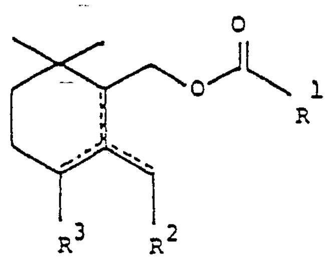

(Ia)

wherein $R^1$, $R^2$ and $R^3$ have the above significance and one of the three lines depicted dotwise represents an additional bond.

6. An odorant substance composition, characterized by a content of a compound of the general formula

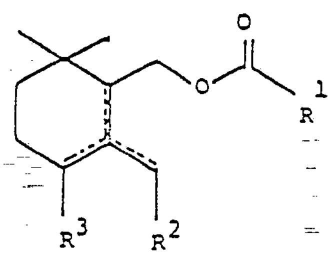

(I)

wherein $R^1$ represents hydrogen, $C_{1-4}$-alkyl or $C_{2-4}$-alkenyl and $R^2$ and $R^3$ represent hydrogen or methyl, but wherein both residues $R^2$ and $R^3$ do not simultaneously represent hydrogen, and one of the three lines depicted dotwise represents an optional additional bond.

7. An odorant substance composition, characterized by a content of 2-ethyl-3,6,6-trimethyl-2-cyclohexene-1-methyl acetate.

8. An odorant substance composition, characterized by a content of a mixture of 2-ethyl-6,6-dimethyl-2-cyclo-hexene-1-methyl acetate and 2,3,6,6-tetramethyl-2-cyclohexene-1-methyl acetate.

9. An odorant substance composition, characterized by a content of a compound of the general formula

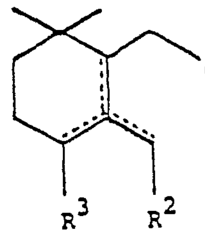

(II)

wherein $R^2$ and $R^3$ represent hydrogen or methyl, but wherein both residues $R^2$ and $R^3$ do not simultaneously represent hydrogen, and one of the three lines depicted dotwise represents an optional additional bond.

10. The use of compounds of the general formula

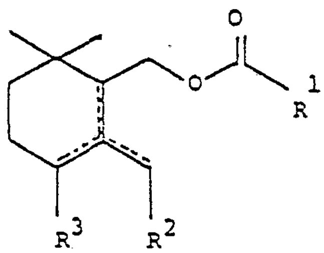

(I)

wherein $R^1$ represents hydrogen, $C_{1-4}$-alkyl or $C_{2-4}$-alkenyl and $R^2$ and $R^3$ represent hydrogen or methyl, but wherein both residues $R^2$ and $R^3$ do not simultaneously represent hydrogen, and one of the three lines depicted dotwise represents an optional additional bond,
as odorant substances or as modifiers of odorant substance compositions.

11. The use of the compounds of the general formula

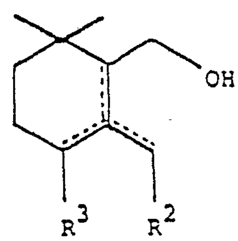

(II)

wherein $R^2$ and $R^3$ represent hydrogen or methyl, but wherein both residues $R^2$ and $R^3$ do not simultaneously represent hydrogen, and one of the three lines depicted dotwise represents an optional additional bond,
as odorant substances or as modifiers of odorant substance compositions.

**Revendications**

1. Composés de formule générale

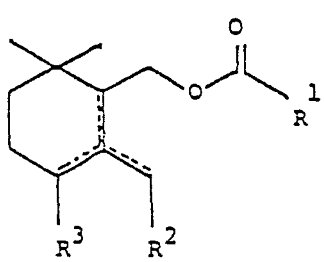

(I)

où $R^1$ représente un hydrogène, un alcoyle en $C_1$ à $C_4$ ou un alcényle en $C_2$ à $C_4$ et $R^2$ et $R^3$ représentent un hydrogène ou un méthyle, mais où les deux radicaux $R^2$ et $R^3$ ne représentent pas simultanément un hydrogène, et l'une des trois lignes représentées en pointillé représente une liaison supplémentaire facultative.

2. 2-éthyl-3,6,6-triméthyl-2-cyclohexén-1-méthyl-acétate.

3. Mélange de 2-éthyl-6,6-diméthyl-2-cyclohexén-1-méthylacétate et de 2,3,6,6-tétraméthyl-2-cyclohexén-1-méthyl-acétate.

4. Composés de formule générale

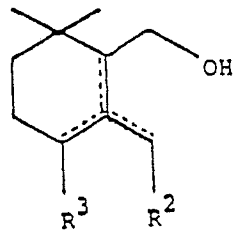

(II)

où $R^2$ et $R^3$ représentent un hydrogène ou un méthyle, mais où les deux radicaux $R^2$ et $R^3$ ne représentent pas simultanément un hydrogène, et l'une des trois lignes représentées en pointillé représente une liaison supplémentaire facultative.

5. Procédé de préparation de composés de formule

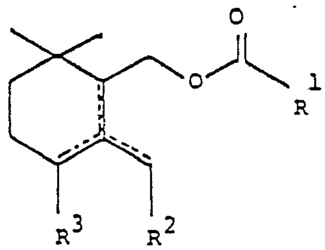

(I)

où $R^1$ représente un hydrogène, un alcoyle en $C_1$ à $C_4$ ou un alcényle en $C_2$ à $C_4$, et $R^2$ et $R^3$ représentent un hydrogène, mais où les deux radicaux $R^2$ et $R^3$ ne représentent pas simultanémlent un hydrogène, et l'une des trois lignes représentées en pointillé représente une liaison supplémentaire facultative,

caractérisé en ce que

a) on estérifie un alcool de formule

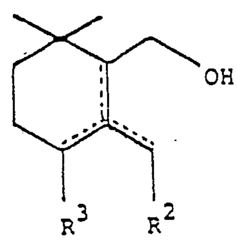

(II)

où $R^2$, $R^3$ et les lignes pointillées ont la signification donnée ci-dessus,

ou

b) on cyclise un ester de formule

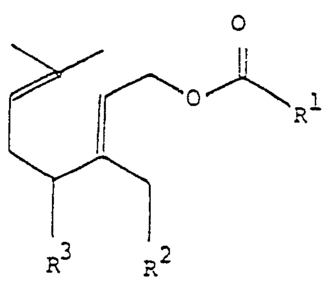

(III)

où $R^1$, $R^2$ et $R^3$ ont les significations données ci-dessus, ou

c) on hydrogène de façon catalytique un composé de formule générale

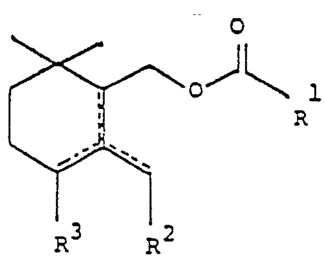

(Ia)

où $R^1$, $R^2$ et $R^3$ ont la signification donnée ci-dessus, et l'une des trois lignes représentées en pointillé représente une liaison supplémentaire.

6. Composition odorante caractérisée en ce qu'elle contient un composé de formule générale

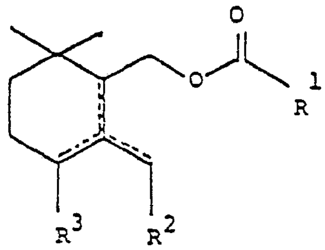

(I)

où $R^1$ représente un hydrogène, un alcoyle en $C_1$ à $C_4$ ou un alcényle en $C_2$ à $C_4$ et $R^2$ et $R^3$ représentent un hydrogène ou un méthyle, mais où les deux radicaux $R^2$ et $R^3$ ne représentent pas simultanément un hydrogène, et l'une des trois lignes représentées en pointillé représente une liaison supplémentaire facultative.

7. Composition odorante caractérisée en ce qu'elle contient le 2-éthyl-3,6,6-triméthyl-2-cyclohexén-1-méthyl-acétate.

8. Composition odorante caractérisée en ce qu'elle contient un mélange de 2-éthyl-6,6-diméthyl-2-cyclohexén-1-méthyl-acétate et de 2,3,6,6-tétraméthyl-2-cyclohexén-1-méthyl-acétate.

9. Composition odorante caractérisée en ce qu'elle contient un composé de formule générale

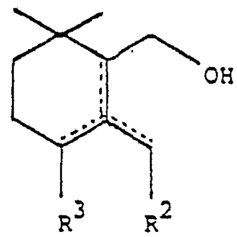

(II)

où $R^2$ et $R^3$ représentent un hydrogène ou un méthyle, mais où les deux radicaux $R^2$ et $R^3$ ne représentent pas simultanément un hydrogène, et l'une des trois lignes représentées en pointillé représente une liaison supplémentaire facultative.

10. Application de composés de formule générale

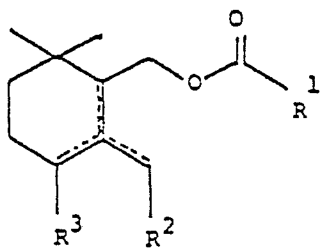

(I)

où $R^1$ représente un hydrogène, un alcoyle en $C_1$ à $C_4$ ou un alcényle en $C_2$ à $C_4$ et $R^2$ et $R^3$ représentent un hydrogène ou un méthyle, mais où les deux radicaux $R^2$ et $R^3$ ne représentent pas simultanément un hydrogène, et l'une des trois lignes représentées en pointillé représente une liaison supplémentaire facultative,
comme produits odorants ou comme modificateurs de compositions odorantes.

11. Application des composés de formule générale

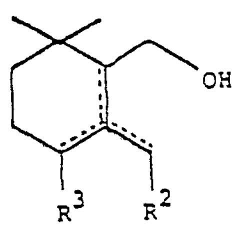

(II)

où $R^2$ et $R^3$ représentent un hydrogène ou un méthyle, mais où les deux radicaux $R^2$ et $R^3$ ne représentent pas simultanément un hydrogène, et l'une des trois lignes représentées en pointillé représente une liaison supplémentaire facultative,
comme produits odorants ou comme modificateurs de compositions odorantes.